(19) Europäisches Patentamt
European Patent Office
Office européen des brevets

(11) **EP 2 412 381 A1**

(12) **EUROPÄISCHE PATENTANMELDUNG**

(43) Veröffentlichungstag:
**01.02.2012 Patentblatt 2012/05**

(51) Int Cl.:
*A61K 38/19* $^{(2006.01)}$    *A61P 9/10* $^{(2006.01)}$

(21) Anmeldenummer: **11008516.4**

(22) Anmeldetag: **12.09.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **13.09.2007  EP 07018029**

(62) Dokumentnummer(n) der früheren Anmeldung(en) nach Art. 76 EPÜ:
**08804077.9 / 2 195 014**

(71) Anmelder: **SYGNIS Bioscience GmbH & Co KG 69120 Heidelberg (DE)**

(72) Erfinder: **Die Erfindernennung liegt noch nicht vor**

(74) Vertreter: **Lahrtz, Fritz**
**Isenbruck Bösl Hörschler LLP**
**Patentanwälte**
**Prinzregentenstrasse 68**
**81675 München (DE)**

Bemerkungen:
Diese Anmeldung ist am 24-10-2011 als Teilanmeldung zu der unter INID-Code 62 erwähnten Anmeldung eingereicht worden.

(54)    **Verwendung von G-CSF für die Behandlung von Schlaganfall**

(57)    Die Erfindung betrifft unter anderem G-CSF zur Verwendung in einem Verfahren zur Behandlung von Schlaganfall beim menschlichen Patienten, wobei das G-CSF dem Patienten in einer Gesamtdosis von 80 bis 150 µg pro kg Körpergewicht über einen Behandlungszeitraum von 2 bis 7 Tagen zu verabreichen ist.

EP 2 412 381 A1

**Beschreibung**

Die Erfindung betrifft die Verwendung von Granulozyten-spezifischen Kolonie stimulierenden Faktor (engl. granulocyte-colony stimulating factor; G-CSF) zur Herstellung eines Arzneimittels für die Behandlung von cerebralem Schlaganfall beim Menschen, wobei das G-CSF in einer täglichen Dosis von 30 bis 180 μg pro kg Körpergewicht über einen Zeitraum vom mindestens 2 Tage zu verabreichen ist.

Cerebraler Schlaganfall ist die dritthäufigste Todesursache und die Hauptursache für Pflegebedürftigkeit in der Welt. Er stellt somit eine enorme sozio-ökologische Belastung dar. Die Ätiologie von Schlaganfall ist entweder ischämisch - wie in den meisten aller Fälle - oder hämorrhagisch. Der ischämische Schlaganfall ist meist embolisch oder thrombotisch verursacht. Bis jetzt gibt es keine effektive Behandlungsmöglichkeit für die Mehrzahl der Schlaganfallpatienten. Die einzigen bis jetzt klinisch zugelassenen Arzeimittel sind der Gewebe-Plasminogenaktivator (engl. tissue plasminogen activator; tPA) und Acetylsalicylsäure. Nach einem massiven Zellsterben im unmittelbaren Kern des Infarkts verursacht durch Glukose- und Sauerstoffinangel (cerebrale Ischämie) vergrößert sich das Infarktgebiet einige Tage lang aufgrund von sekundären Mechanismen wie z.B. Glutamat-Exocitoxizität, inflammatorische Mechanismen, Erzeugung von freien Radikalen und apoptotischen Mechanismen (Leker & Sholzami, Brain Res. Rev. 2002; 39: 55-73). Das Infarktvolumen kann mittels Kernspintomographie bestimmt werden; mit der DWI-Methode ("diffusion weighted image") wird initial das zellulär bereits geschädigte bzw. zerstörte Areal bestimmt, während die PWI-Methode ("perfusion weighted image"), bei der die Verteilung eines Kontrastmittels untersucht wird, Aufschluss gibt über die Größe des aktuell unzureichend durchbluteten Gewebeareals. Häufig ist das mittels PWI-bestimmte Gewebeareal größer als das DWI-bestimmte. In diesen Fällen wird davon ausgegangen, dass durch eine Wiederherstellung der Durchblutung (z.B. durch thrombolytische Behandlung mit tPA) der Teil des PWI-bestimmten Gewebeareal, der nicht mit dem DWI-bestimmten Areal überlappt, funktional erhalten werden kann, wohingegen das DWI-bestimmte Gewebeareal möglicherweise weiniger gut gerettet werden kann (Beaulieu et al. Ann Neurol 1999; 46:558-578; Wu et al. Stroke 2001; 32:933-942).

Mit den bislang zugelassenen Medikamente kann eine Wiederherstellung der Durchblutung bei einem ischämischen Schlaganfall erreicht werden, wenn die Behandlung rechtzeitig (maximal etwa 3 bis 6 h nach dem Schlaganfall) einsetzt. Eine protektive Wirkung auf die vom Schlaganfall betroffenen Neuronen (Neuroprotektion) oder gar eine die Neubildung von Neuronen im betroffenen Bereich fördernde Eigenschaft (Neuroregeneration) besitzen diese Medikamente nicht. Dementsprechend besteht ein starker Bedarf an neuen, vor allem neuroprotektiven und/oder neuroregenerativen Behandlungsmöglichkeiten zur Verbesserung des klinischen Ausgangs von cerebralem Schlaganfall. Solche neuen Behandlungsmöglichkeiten sollten bevorzugt auch geeignet sein für cinc crst spät einsetzende Behandlung von Schlaganfall, z.B. eine erst 6 h und später einsetzende Behandlung.

Für die quantitative Erfassung der Schwere eines Schlaganfall, akut und unter einer Behandlung, werden im Allgemeinen Bewertungsskalen wie die modifizierte Rankin-Skala oder die NIH-Schlaganfall-Skala (engl. NIH stroke scale, NIHSS) verwendet. Während die Rankin-Skala eine recht grobe Einteilung des neurologischen Zustands eines Patienten ermöglicht (zwischen dem Wert "0" für "frei von Symptomen" und dem Wert "6" für "tot") erlaubt die N1H-Schlaganfall-Skala eine relativ fein aufgelöste Bewertung des neurologischen Zustands eines Patienten. Bei der NIH-Schlaganfall-Skala werden für die Erhebung des Befunds verschiedene neurologische Aspekte untersucht und mit Punkten bewertet. Die Gesamtzahl der Punkte gibt ein Maß für die Schwere der Schlaganfallsymptomatik, wobei die Punktzahl mit der Schwere der Symptomatik ansteigt. Diese Bewertungsskalen sind auch geeignet, den Verlauf der Symptomatik nach einem Schlaganfall zu überwachen und den Erfolg einer eingesetzten Therapie zu quantifizieren. Im Allgemeinen lässt sich eine Korrelation zwischen der Infarktgröße und der Quantifizierung der Schlaganfallschwere mittels Schlaganfall-Skala feststellen (Beaulieu et al. Ann Neurol 1999; 46:568-578). Von daher ist auch der Verlauf der Infarktgröße unter der Behandlung geeignet zur Beurteilung eines Behandlungseffekts.

G-CSF gehört zu der Gruppe der Kolonie stimulierende Faktoren (CSF). Dabei handelt es sich um regulatorische Proteine, die verantwortlich sind für Kontrolle der Proliferation und Differenzierung von hämatopoetischen Zellen wie Granulozyten, Megakaryozyten und Monozyten bzw. Makrophagen. Ohne entsprechende CSFs können diese hämatopoetischen Zellen in Kultur nicht überleben bzw. proliferieren. Die CSFs gehören zu der Gruppe der Zytokine. Zusammen mit Erythropoetin (EPO) und einigen Interleukinen bilden sie die Gruppe der hämatopoetischen Wachstumsfaktoren.

Die Gruppe der CSFs umfasst im einzelnen die Faktoren M-CSF (Makrophagenspezifischer Kolonie stimulierender Faktor; auch CSF-1), GM-CSF (Makrophagen/Granulozyten-spezifischer Kolonie stimulierender Faktor; auch CSF-2), G-CSF (Granulozyten-spezifischer Kolonie stimulierender Faktor; auch CSF-3) und multi-CSF (multifunktioneller Kolonie stimulierender Faktor; auch IL3) entsprechend ihrer Spezifität hinsichtlich der verschiedenen hämatopoetischen Zellen. Die Reinigung und Klonicrung der einzelnen CSFs ermöglichte eine molekulare Charakterisierung. Bei den genannten vier CSFs handelt es sich um Glykoproteine, allerdings weisen sie auf der Ebene der Primärstruktur (Aminosäure-Sequenz) keinerlei Homologie auf (Metcalf, Cancer 1990; 65:2185-2194; Pimentel, Ann. Clin. Lab. Sc. 1990; 20:36-55).

G-CSF wird sezerniert von aktivierten Monozyten, Makrophagen und Neutrophilen, von Stromazellen, Fibroblasten und Endothelzellen, sowie von verschiedenen Tumor-Zelllinien (z.B. humane Blasenkarzinom-Zelllinie). Reifes humanes G-CSF ist ein monomeres Glykoprotein mit 174 Aminosäuren, wobei der Zuckeranteil für die biologische Aktivität nicht nötig ist. Eine weitere, durch alternatives Splicing der RNA hervorgehende, Variante mit 177 Aminosäuren weist eine deutlich reduzierte biologische Aktivität *auf (*Nagata, BioEssays 1989; 10.113-117*).*

G-CSF fördert die Proliferation und Differenzierung von hämatopoetischen Vorläuferzellen hin zu neutrophilen Granulozyten und aktiviert diese auch. Darüber hinaus wirkt G-CSF auch als Mitogen.

Angesichts dieser Förderung von Proliferation, Differenzierung und Aktivierung von Zellen des hämatopoetischen Systems ist G-CSF zugelassen für die Behandlung von Neutropenie, z.B. in Folge von Chemo- und/oder Strahlentherapie. Darüber hinaus wird G-CSF klinisch verwendet, um die Produktion von Neutrophilen im Knochenmark zu stimulieren, etwa im Vorfeld einer Knochenmarks-Spende bei einer Knochenmarkstransplantation. Seit einigen Jahren ist G-CSF auch für die Behandlung von Neutropenie im Rahmen einer HIV-Erkrankung zugelassen. In der Therapie findet vor allem rekombinantes G-CSF (z.B. Filgrastim, Neupogen(R)) Verwendung. Für die Behandlung von Neutropenie im Rahmen einer Chemo- und/oder Strahlentherapie werden üblicherweise Tagesdosen von etwa 5 $\mu$g (entsprechend 0,5 Mio. Einheiten) pro kg Körpergewicht eingesetzt. Die Dosis wird üblicher Weise verabreicht als subcutane Bolus-Injektion, als kontinuierliche subcutane Injektion, als kurzzeitige intravenöse Injektion (binnen von 15 bis 30 min) oder als kontinuierliche intravenöse Injektion. Bei der Neutropenie-Behandlung im Rahmen einer HIV-Infektion ist die eingesetzte Dosis im Allgemeinen deutlich geringer als bei der im Rahmen von Chemo- und/oder Strahlentherapie.

Neuere Forschungsarbeiten zeigen, dass G-CSF neben seiner Leukozyten stimulierenden Wirkung auch noch weitere klinisch relevante Eigenschaften aufweist. So wurde z.B. die Verwendung von G-CSF und anderen CSF zur Behandlung von Infektionen (*WO 88/00832),* zur Förderung der Wundheilung *(WO 92/14480)* und zur Stimulation von Angiogenese (*WO 97/14307*) beschrieben. Takeshi und Yoshihiro wiederum beschrieben, dass G-CSF und andere Faktoren geeignet sind, die Acetylcholin-Transferase (ChAT) zu aktivieren und so bei verschiedenen neurodegenerativen Erkrankungen (z.B. Alzheimers Erkrankung und Demenz) beitragen können zur Verlängerung des Überlebens der betroffenen Zellen *(*JP 03537151*).*

Buschmann und Scharper beschrieben, dass G-CSF und GM-CSF arteriogen wirken, also das Wachstum von kollateralen Arterien aus bereits bestehenden arteriolaren Verbindungen verstärken *(*EP 1019082*).* Auf dieses Weise können diese Faktoren beitragen, die Wiederdurchblutung von ischämischen Gewebe, u.a. bei cerebralem Schlaganfall, zu verbessern (Buschmann et al. Circulation 2003; 108:610-615*).* Darüber hinaus wird beschrieben, dass G-CSF durch die Stimulierung der Mobilisierung von Knochenmarksstammzellen geeignet ist, die Neuroregeneration von geschädigtem Nervengewebe nach Schlaganfall oder anderen neurodegenerativen Erkrankungen zu fördern (WO 02/099081, EP 1465653*).*

Die vor kurzem gemachte Beobachtung, dass G-CSF-Rezeptoren auch auf Neuronen zu finden sind (DE 10033219*),* legt nahe, dass G-CSF auch direkt auf diese Zellen des ZNS wirken kann. Entsprechend wurde kürzlich im Tiermodell für die Behandlung von fokaler cerebraler Ischämie eine neuroprotektive und neuroregenerative Wirkung für G-CSF gezeigt *(*Schabitz et al. Stroke 2003; 34: 745-751, Schneider et al. J Clin Invest 2005: 115:2083-2098, *WO 2004/58287; wo 2006/08582).*

Ziel der vorliegenden Erfindung ist es G-CSF als Arzneimittel zu Behandlung von Schlaganfallpatienten in einer Dosis, einem Dosis-Regime und einer Darreichungsform anzubieten, die eine besonders geeignete Effektivität aufweisen, ohne dass es zu ungünstigen Nebenwirkungen kommt.

Entsprechend betrifft die hier dargestellte Erfindung die Verwendung von G-CSF für die Herstellung eines Arzneimittels zur Behandlung von Schlaganfall beim menschlichen Patienten, wobei das G-CSF dem Patienten in einer Gesamtdosis von etwa 30 bis 180 $\mu$g pro kg Körpergewicht über einen Zeitraum von 2 bis 7 Tagen zu verabreichen ist.

Die Erfindung betrifft ferner G-CSF zur Verwendung in einem Verfahren zur Behandlung von Schlaganfall beim menschlichen Patienten, wobei das G-CSF dem Patienten in einer Gesamtdosis von etwa 30 bis 180 $\mu$g pro kg Körpergewicht über einen Zeitraum von 2 bis 7 Tagen zu verabreichen ist.

Die Erfindung betrifft ferner ein Verfahren zur Behandlung von Schlaganfall in einem menschlichen Patienten, wobei G-CSF dem Patienten in einer Gesamtdosis von etwa 30 bis 180 $\mu$g pro kg Körpergewicht über einen Zeitraum von 2 bis 7 Tagen zu verabreichen ist.

Im Rahmen der vorliegenden Erfindung werden die Begriffe "Patient" und "Patienten" austauschbar verwendet und betreffen sowohl den Singular als auch den Plural. Ferner werden auch die Begriffe "Patient" und "Schlaganfallpatient" austauschbar verwendet.

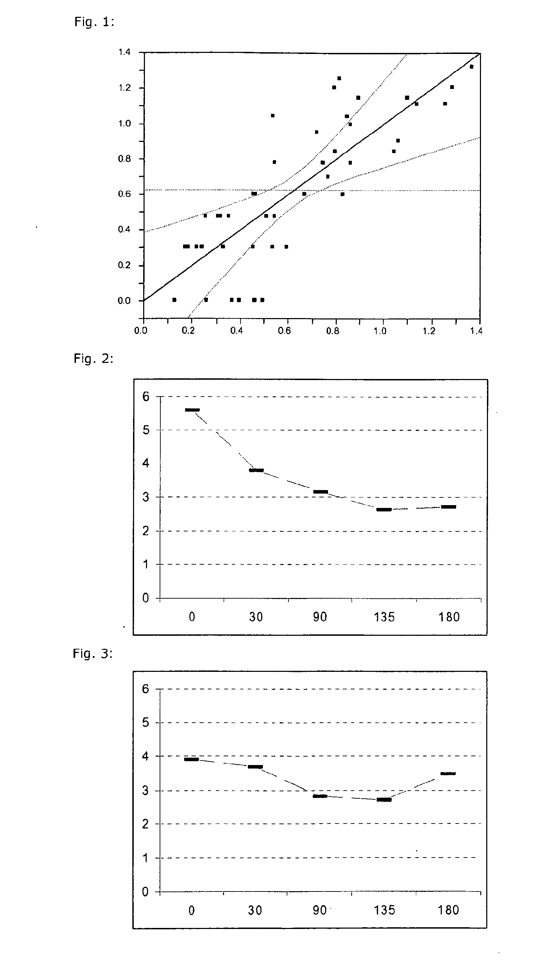

Die Dosisstufen von 90 $\mu$g und vor allem 135 $\mu$g G-CSF Gesamtdosis pro kg Körpergewicht sind für die Behandlung von Schlaganfallpatienten besonders geeignet, wie die im Beispiel 1 beschriebene Studienauswertung unerwarteter Weise ergibt. Eine weitere Erhöhung der Gesamtdosis über die Dosisstufe von 135 $\mu$g pro kg Körpergewicht hinaus verbesserte den Behandlungserfolg nicht. Bei Patienten mit mittleren initialen Infarktgrößen von etwa 25 cm$^3$ (DWI-bestimmt) führte dieser Studienauswertung zufolge die höhere Dosisstufe von 180 $\mu$g Gesamtdosis pro kg Körpergewicht sogar zu einem geringeren Behandlungserfolg als bei der Dosisstufe von 135 $\mu$g pro kg Körpergewicht (Fig. 3). Niedrigere Dosierungen, wie sie z.B. bei Neutropenie-Behandlungen eingesetzt werden (30 $\mu$g pro kg Körpergewicht oder weniger) führten der Studienauswertung zufolge zu keinem oder nur zu suboptimalen Behandlungserfolg (Fig. 2 und 3). Da bei der Schlaganfallbehandlung das Gewicht der Patienten nur geschätzt wird, ist davon auszugehen, dass die tatsächlich verabreichte Gesamtdosis mit einer Toleranz von etwa 10 % um den vorgesehenen Wert der Dosisstufe schwankt. Demnach entspricht die Dosisstufe von 90 µg pro kg Körpergewicht einer tatsächlich verabreichten Gesamtdosis von 80 bis 100 µg pro kg Körpergewicht und die Dosisstufe von 135 µg pro kg Körpergewicht einer tatsächlich verabreichten Gesamtdosis von 120 bis 150 µg pro kg Körpergewicht.

Bevorzugt ist daher eine Gcsamtdosis von 80 bis 150 µg G-CSF pro kg Körpergewicht einzusetzen (entsprechend den Dosisstufen von 90 µg G-CSF Gesamtdosis pro kg Körpergewicht bis 135 µg G-CSF Gesamtdosis pro kg Körpergewicht bei einer Toleranz von etwa 10 % hinsichtlich der tatsächlich verabreichten Gesamtdosis pro kg Körpergewicht), die verteilt über einen Zeitraum von 3 Tagen intravenös zu verabreichen ist.

Besonders bevorzugt ist weiter eine Gesamtdosis von 120 bis 150 µg G-CSF pro kg Körpergewicht einzusetzen (entsprechend der Dosisstufen von 135 µg G-CSF Gesamtdosis pro kg Körpergewicht bei einer Toleranz von etwa 10 % hinsichtlich der tatsächlich verabreichten Gesamtdosis pro kg Körpergewicht), die verteilt über einen Zeitraum von 3 Tagen intravenös zu verabreichen ist. Ganz besonders bevorzugt ist eine Dosis von 135 µg G-CSF pro kg Körpergewicht einzusetzen.

In einer erfindungsgemäßen Ausführungsform ist zunächst ein Anteil von 20 bis 50 %, bevorzugt ein Anteil von einem Drittel an der Gesamtdosis bei Aufnahme der Behandlung als Bolus schnell (z.B. binnen von etwa 20 min) intravenös zu injizieren, während der restliche Anteil zur Aufrechterhaltung eines gleichmäßig hohen Serumspiegels kontinuierlich über einen Zeitraum von 2 bis 7 Tagen, bevorzugt von 3 Tagen, intravenös zu verabreichen ist.

Gemäß der Auswertung der in Beispiel 1 beschriebenen Studie ergibt sich für die Verabreichung einer G-CSF Gesamtdosis ohne Berücksichtigung des Körpergewichts der Patienten eine vergleichbare Abhängigkeit des Behandlungserfolgs von der eingesetzten G-CSF Gesamtdosis. Alternativ ist daher erfindungsgemäß auch eine G-CSF-Gesamtdosis von 2 bis 16 mg (entsprechend der Dosisstufen von 30 µg G-CSF Gesamtdosis pro kg Körpergewicht bis 180 µg G-CSF Gesamtdosis pro kg Körpergewicht), bevorzugt 6 bis 12 mg (entsprechend der Dosisstufen von 90 µg G-CSF Gesamtdosis pro kg Körpergewicht bis 135 µg G-CSF Gesamtdosis pro kg Körpergewicht), besonders bevorzugt 9,5 bis 12 mg (entsprechend der Dosisstufen von 135 µg G-CSF Gesamtdosis pro kg Körpergewicht) ohne Berücksichtigung des Körpergewichts des jeweiligen Patienten für die Behandlung einzusetzen.

Dic im Rahmen dieser Erfindung für die Behandlung von Schlaganfallpaticntcn eingesetzte G-CSF-Gesamtdosisbereich von 30 bis 180 µg pro kg Körpergewicht über einen Zeitraum von 3 Tagen (Beispiel 1) geht deutlich hinaus über die G-CSF-Dosen, wie sie für die bislang zugelassenen Indikationen eingesetzt werden. Dennoch wurde die ertindungsgemäße Behandlung von den Schlaganfallpatienten gut vertragen und führte zu keinen sicherheitsrelevanten Nebenwirkungen.

Die erfindungsgemäße Behandlung mit dem neuroregenerative wirkenden G-CSF erlaubt einen vergleichsweise späten Beginn der Behandlung nach dem Schlaganfall, verglichen z.B. mit der auf thrombolytischer Wirkung beruhenden tP A-Behandlung, die nur für einen Behandlungsbeginn bis 3 h nach dem Schlaganfall zugelassen ist. Entsprechend hatten die in die erfindungsgemäße Studie eingeschlossenen Patienten den Schlaganfall zwischen 4 und 18 h vor Behandlungsbeginn. Im Mittel erfolgte der Behandlungsbeginn etwa 10 h nach dem Schlaganfall.

Die statistische Auswertung dieser Studie (Beispiel 1) ergab eine Wirksamkeit der erfindungsgemäßen Verabreichung von G-CSF bei Infarkten mit einer initialen Größe (DWI-bestimmt) von 16 cm$^3$ und mehr.

Weiter wurden überraschender Weise gefunden, dass die erfindungsgemäße Verabreichung von G-CSF besonders geeignet ist zur Behandlung von schwereren Schlaganfällen, die ein vergleichsweise großes Infarktvolumen haben. Während der Effekt der erfindungsgemäßen Behandlung bei kleinen Infarkten nur gering ausfällt, kam es bei der erfindungsgemäßen Behandlung von großen Infarkten zu einem großen Effekt. Entsprechend ist die erfindungsgemäße Verabreichung von G-CSF bevorzugt einzusetzen zur Behandlung von Infarkten mit einem Infarktvolumen von min. 16 cm$^3$ ((DWI-bestimmf). bevorzugt min. 25 cm$^3$ (DWI-bestimmt; Fig. 3)₎ ganz besonders bevorzugt zur Behandlung von Infarkten mit einem Infarktvolumen von min. 50 cm$^3$ (DWI-bestimmt, Fig. 2).

Gemäß einer bevorzugten Ausführungsform der erfindungsgemäßen Verwendung werden daher die die Patienten vor der Behandlung darauf getestet, ob sie einen Schlaganfall mit einem initialen DWI-bestimmten Infarktvolumen von dem jeweiligen Mindestvolumen (d.h. 16 cm$^3$ 25 cm$^3$ oder 50 cm$^3$) aufweisen.

Daher betrifft die Erfindung auch cin Verfahren zur Identifikation von Schlaganfallpatienten, die ansprechen auf eine Behandlung umfassend die Verabreichung von G-CSF, und umfassend die Schritte

    a. DVdl-basierte Bestimmung des Infarktvolumens, und
    b. Identifikation der Patienten mit einem Infarktvolumen von min. 16 cm$^3$ als Kandidaten für besagte Behandlung.

Ferner betrifft die Erfindung daher auch ein Verfahren zur Identifikation von Schlaganfallpatienten, die ansprechen auf eine Behandlung umfassend die Verabreichung von G-CSF, und umfassend die Schritte

    a. DVhI-basierte Bestimmung des Infarktvolumens, und

b. Identifikation der Patienten mit einem Infarktvolumen mit Beteiligung des corticalen Hirngewebes als Kandidaten für besagte Behandlung.

Diese erfindungsgemäße Verfahren ist besonders nützlich um Patienten zu ermitteln, die auf die erfindungsgemäße Verwendung von G-CSF besonders ansprechen. Es kann aber auch allgemein zur Ermittlung von Patienten herangezogen werden, die allgemein auf eine G-CSF Behandlung besonders ansprechen.

Gemäß einer bevorzugten Ausführungsform der Erfindung erfolgt die Verabreichung von G-CSF wie im Rahmen der erfindungsgemäßen Verwendung definiert.

Während die kleinen Infarkten bei dieser Studie hauptsächlich sub-cortical lokalisiert sind, weisen die größeren Infarkte in der Regel eine Beteiligung von corticatem Hirngewebe auf. Je größer der Infarkt ist, desto stärker ist auch die Schädigung von corticalem Hirngewebe. Demnach ist davon auszugehen, dass das corticale Hirngewebe einer Behandlung mit G-CSF in besonderer Weise zugänglich ist. Entsprechend ist die erfindungsgemäße Verabreichung von G-CSF bevorzugt einzusetzen zur Behandlung von Infarkten mit einer Beteiligung von corticalem Hirngewebe.

Der Wirkstoff G-CSF kann für die erfindungsgemäße Verabreichung mit einem oder mehreren pharmazeutisch verträglichen Hilfsstoffen formuliert und kombiniert werden. Die Bezeichnung "pharmazeutisch verträglich" bezieht sich auf Moleküle und Zusammensetzungen, die physiologisch toleriert werden, und nicht typischerweise allergische oder unerwünschte Reaktionen wie Schwindelaiifälle hervorrufen.

Die Bezeichnung "Hilfsstoffe" bezieht sich auf ein Verdünnungsmittel, Adjuvanz, Arzneiträger oder sonstige Hilfsmittel mit dem der Wirkstoff zu verabreichen ist. Bei solchen pharmazeutischen Hilfsstoffen kann es sich um sterile Flüssigkeiten, wie Wasser, Salinelösungen, Pufferlösungen, Dextroselösungen, Glycerollösungen, Detergentienlösungen DMSO oder Wasser- und Öl-Emulsionen handeln. Wasser, Salinelösungen, Pufferlösungen, Dextroselösungen und Glycerollösungen werden bevorzugt als Hilfsstoffe eingesetzt, besonders für zu injizierende Lösungen des Wirkstoffs. Besonders bevorzugt ist die Verwendung von G-CSF als Wirkstoff in Kombination mit den Hilfsstoffen Natriumacetat-Puffer mit einem pH-Wert von 4, Sorbitol und dem Detergenz Tween-80, sowie einer Dextroselösung.

Mit "Behandlung" ist das Verzögern, Unterbrechen, Anhalten, Umkehren oder Stoppen des Fortschreitens des Zustandes nach dem Schlaganfall gemeint, was nicht notwendigerweise die vollständige Beseitigung aller Schlaganfallanzeichen und - symptome erfordert. Darüber hinaus ist es nicht notwendig, dass die Behandlung bei 100 % der behandelten Patienten Effektivität zeigt, vielmehr ist mit dem Terminus "Behandlung", gemeint, dass ein statistisch signifikanter Anteil der Patienten effektiv behandelt werden kann, derart dass die Symptome und klinischen Anzeichen zumindest verbessert werden. Ob der Anteil statistisch signifikant ist, kann vom Fachmann ohne weiteres mittels verschiedener statistischer Verfahren (z.B. Konfidenzintervalle, p-Wert-Bestimmung, Student's t-Test, Mann Whitney Test, usw.) bestimmt werden. Bevorzugte Konfidenzintervalle haben eine Konfidenz von min. 90 %, min. 95 %, min. 97 %, min. 98 % oder min. 99 %. Die p-Werte sind bevorzugt 0,1, 0,05, 0,01, 0,005 oder 0,0001.

Unter "Effekt" bzw. "Effektivität" bzw. "Wirksamkeit" ist im Rahmen dieser Erfindung das Ausmaß eines Behandlungserfolges zu verstehen, bestimmt z.B. anhand der Verbesserung der klinischen Anzeichen und Symptome. Geeignete Beurteilungskriterien für solche Verbesserungen im Rahmen einer Schlaganfallbehandlung sind z.B. aber nicht abschließend die Infarktgröße oder neurologische Bewertungsskalen wie die NIH-Schlaganfall-Skala oder die modifizierte Rankin-Skala.

In weiteren Ausführungsformen kann die erfindungsgemäße Schlaganfallbehandlung kombiniert werden mit der Verabreichung von einem oder mehr zusätzlichen Faktoren. "Zusätzliche Faktoren" gemäß der Erfindung meint jegliche Substanz, die den Effekt der erfindungsgemäßen Behandlung von Schlaganfall mit G-CSF unterstützt. Geeignete zusätzliche Faktoren sind z.B. Faktoren mit neuroprotektiver Wirkung wie Erythropoietin, BDNF, VEGF, CNTF, GM-CSF oder inflammationsmodulierende Faktoren. Die zusätzliche Gabe von Bradykinin oder analogen Substanzen bei einer intravenösen Verabreichung kann die Zufuhr der Wirkstoffe zum Gehirn unterstützen (Emerich et al., Clin Pharmacokinet 2001; 40:105-123; Siegal et al., Clin Pharmacokinet 2002; 41:171-186). Antiapoptotische Agenzien oder Agenzien, die die Passage durch die Blut-Hirn-Schranke erleichtern können ebenfalls verwendet werden. Die Verabreichung der zusätzlichen Faktoren kann gleichzeitig, vor oder nach der erfindungsgemäßen Verabreichung des G-CSF erfolgen.

Die Erfindung wird darüber hinaus durch die Abbildungen näher erläutert.

Die anliegenden Abbildungen zeigen in:

Fig. 1 Korrelation für das in Beispiel 1 beschriebene lineare Modell. Für die einzelnen Patienten ist die Zielparameter in Abhängigkeit von den in das Modell eingehenden Kenngrößen aufgetragen. Der Konrelationskoeffizient für das Modell beträgt 0.67 und der p-Wert für das Modell ist kleiner 0,0001.

Fig. 2 Dosis-Wirkungs-Kurve für große Infarkte. Aufgetragen ist der aus dem statistischen Modell von Beispiel 1 geschätzte Behandlungserfolg (angegeben als Wert der NIH-Schlaganfall-Skala 90 Tage nach dem Behandlungsbeginn) in Abhängigkeit von der zu verabreichenden G-CSF Dosisstufe (in μg pro kg Körpergewicht, wobei "0" der Behandlung mit Plazebo entspricht). Die Dosis-Wirkungs-Kurve ist aus dem statistischen Modell abgeleitet für Patienten mit einem inititalen Wert von 8.65 nach der NIH-Schlaganfall-Skala, einem Alter von etwa 70 Jahren und einem initialen (DWI-bestimmten) Infarktvolumen von etwa 50 cm$^3$.

Fig. 3 Dosis-Wirkungs-Kurve für mittlere Infarkte. Aufgetragen ist der aus dem statistischen Modell von Beispiel 1 geschätzte Behandlungserfolg (angegeben als Wert der NIH-Schlaganfall-Skala 90 Tage nach dem Behandlungsbeginn) in Abhängigkeit von der zu verabreichenden G-CSF Dosisstufe (in $\mu$g pro kg Körpergewicht, wobei "0" der Behandlung mit Plazebo entspricht). Die Dosis-Wirkungs-Kurve ist aus dem statistischen Modell abgeleitet für Patienten mit einem inititalen Wert von 8.65 nach der NIH-Schlaganfall-Skala, einem Alter von etwa 70 Jahren und einem initialen (DWI-bestimmten) Infarktvolumen von etwa 25 cm$^3$.

Beschreibung des Ausführungsbeispiels:

Beispiel 1:

Die Sicherheit und Wirkung der Verwendung von G-CSF zur Behandlung von cerebralem Schlaganfall wurde in einer Placebo-kontrollierten Doppelblindstudie mit eskalierenden Dosisstufen nach Maßgabe der Ethikkommission untersucht. In die Studie aufgenommen wurden insgesamt 43 Patienten. 14 Patienten erhielten Placebo (Gruppe P), während jeweils 7 Patienten eine G-CSF Gesamtdosis von 30 $\mu$g pro kg Körpergewicht (Gruppe I), 90 $\mu$g pro kg Körpergewicht (Gruppe II) bzw. 180 $\mu$g pro kg Körpergewicht (Gruppe IV) verabreicht wurde. 8 Patienten erhielten eine G-CSF Gesamtdosis von 135 $\mu$g pro kg Körpergewicht (Gruppe III).

Eingeschlossen wurden männliche und weibliche Patienten in einem Alter von 40 bis 87 Jahren, mit einem akuten cerebralen Schlaganfall zwischen etwa 4 bis 18 h vor Beginn der Behandlung. Im Mittel erfolgte der Behandlungsbeginn etwa 10 h nach dem Infarkt. Die in die Studie eingeschlossenen Patienten hatten eine initiale DWI-bestimmte Infarktgröße zwischen etwa 1 und 100 cm$^3$ und einer initialen Bewertung nach der NIH-Schlaganfall-Skala zwischen 1 und 19. Außerdem gehörte zu den Einschlusskriterien, dass das initiale PWI-bestimmte Infarktareal der Patienten das größer seien sollte als das initiale DWI-bestimmte Infarktareal (DWI/PWI-Mismatch).

Den Patienten wurde das Placebo bzw. der Wirkstoff intravenös über einen Zeitraum von 3 Tagen ab Behandlungsbeginn verabreicht, wobei ein Drittel der Gesamtdosis zu Bchandlungsbeginn als Bolus in einer etwa 20 min Infusion verabreicht wurde. Die restlichen Zweidrittel der Gesamtdosis wurden anschließend in einer gleichmäßigen Infusion über den gesamten Behandlungszeitraum verabreicht, um einen gleichmäßig hohen Serumspiegel zu gewährleisten. Als Wirkstoff wurde rekombinant hergestelltes G-CSF (Neupogen(R)) in dem entsprechenden Standrad-Puffer (10 mM Natriumactat-Puffer mit einem pH-Wert von 4, 50 mg/ml Sorbitol und 0.004 % Tween-80) eingesetzt, das für die Infusion in Dextroselösung verdünnt wurde.

In keiner der eingesetzten Dosisstufen wurden sicherheitsrelevante Nebenwirkungen durch die erfindungsgemäße G-CSF-Behandlung bei den Schlaganfallpatienten festgestellt.

Zur Beurteilung des Behandlungserfolgs erfolgte 90 Tage nach Behandlungsbeginn eine Beurteilung des neurologischen Zustands der Schlaganfallpatienten entsprechend der NIH-Schlaganfall-Skala.

Vor Entblindung der Studie wurden Kenngrößen bestimmt, die offensichtlich signifikanten Einfluss auf den Behandlungserfolg hatten. Basierend auf den folgenden Kenngrößen wurde ein lineares Modell gebildet:

A: Alter des Patienten
No: Wert auf der NIH-Schlaganfall-Skala zu Behandlungsbeginn
V: DWI-bestimmte Infarktgröße zu Behandlungsbeginn in logarithmischer Skalierung
D: verabreichte G-CSF Gesamtdosis

Die Interaktion zwischen der verabreichte G-CSF Gesamtdosis (D) und DWI-bestimmte Infarktgröße zu Behandlungsbeginn in logarithmischer Skalierung (V) wurde als zusätzlicher Parameter (D*V) ebenfalls in das Modell aufgenommen.

Als Maß für den Behandlungserfolg (Zielparameter Y) diente der Logarithmus der Bewertung nach der NIH-Schlaganfall-Skala zum Behandlungsende nach 90 Tagen.

Das lineare Modell hatte damit die folgende Form:

$$Y = a_1 * D + a_2 * V + a_3 * D*V + a_4 * A + a_5 * N_0 + \varepsilon$$

wobei ε der Restfehlerterm ist.

Die Parameter $a_1$ bis $a_5$ wurden nach erfolgter Entblindung mit Hilfe geeigneter statistischer Verfahren aus den Patientendaten bestimmt.

Fig. 1 zeigt die Korrelation zwischen den eingehenden Parametern und dem Ergebnisparameter entsprechend dem gefundenen linearen Modell. Das lineare Modell weist mit einem Korrelationskoeffizient $r^2=0{,}67$ und einem p-Wert von <0.0001 eine gute Korrelation auf.

Auf Basis dieses Modells lassen sich die zu erwartenden Behandtungsergebnisse (in Form des NIH-Schlaganfall-Skalen-Wertes nach 90 Tagen) für Behandlungen mit Placebo bzw. den verschiedenen G-CSF-Dosisstufen in Abhängigkeit von dem Patientenalter, seinem neurologischen Zustand zu Beginn der Behandlung (NIH-Schlaganfall-Skala) und seiner initialen Infarktgröße (DWI-bestimmt) abschätzen. Das Patientenalter und sein initialer neurologischer Zustand wirken sich zwar deutlich auf die allgemeine Prognose aus (bei jüngeren Patienten und solchen mit nur leichten initialen neurologischen Defiziten ist auch eine allgemein gute Besserung der neurologischen Symptome über den Beobachtungszeitraum von 90 Tagen zu erwarten), sie haben aber in diesem statistischen Modell keinen Einfluss auf die prinzipielle Gestalt der Dosis-Wirkungskurve, also auf den Unterschied des Behandlungserfolgs zwischen einer Placebo- und einer erfindungsgemäßen G-CSF-Behandlung. Dagegen hat entsprechend diesem statistischen Modell die initiale Infaktgröße (DWI-bestimmt) einen deutlichen Einfluss auf das Ausmaß des G-CSF-Behandlungserfolgs im Vergleich zu der Placebo-Behandlung. Auf Basis des statistischen Modells ergibt sich für Infarkte mit einer initialen Größe (DWI-bestimmt) von 16 cm$^3$ und mehr eine Wirksamkeit der erfindungsgemäßen Verabreichung von G-CSF. Weiter lässt sich auf Basis des statistischen Modells abschätzen, dass mit der erfindungsgemäßen G-CSF-Behandlung (vor allem für die G-CSF Gesamtdosis von 135 µg pro kg Körpergewicht) im Vergleich zu der Placebo-behandlung eine besonders deutliche Verbesserungen der neurologischen Symptomatik (beurteilt nach NIH-Schlaganfall-Skala nach 90 Tagen) zu erwarten ist für Patienten mit einem relativ großen Infarktvolumen von etwa 50 cm$^3$ (Fig. 2) und größer. Für Patienten mit einer mittleren Infarktgröße von etwa 25 cm$^3$ ist immer noch eine deutlich bessere neuronale Symptomatik nach erfindungsgemäßer G-CSF-Behandlung im Vergleich zu einer Placebo-Behandlung zu erwarten (Fig. 3). Bei kleineren Infarkten ist gemäß dieser statistischen Betrachtung nur ein geringer Behandlungserfolg durch die G-CSF-Behandlung zu erwarten.

Die aus dem statistischen Modell geschätzten Dosis-Wirkungs-Kurven (Fig. 2 und Fig. 3) zeigen, dass eine optimale Gesamtdosis für die erfindungsgemäße G-CSF-Behandlung bei etwa 135 µg pro kg Körpergewicht zu erwarten ist. Eine weitere Erhöhung der G-CSF-Gcsamtdosis lässt keine weitere Verbesserung des Behandlungserfolgs erwarten.

Die folgenden Seiten 15 und 16 enthalten spezielle Ausführungsformen.

1. Verwendung von G-CSF für die Herstellung eines Arzneimittels zur Behandlung von Schlaganfall beim menschlichen Patienten, wobei das G-CSF dem Patienten in einer Gesamtdosis von 80 bis 150 µg pro kg Körpergewicht über einen Behandlungszeitraum von 2 bis 7 Tagen zu verabreichen ist.

2. Die Verwendung gemäß 1, wobei die zu verabreichende Gesamtdosis zwischen 120 und 150 µg pro kg Körpergewicht liegt.

3. Die Verwendung gemäß 1 oder 2, wobei die Gesamtdosis über einen Behandlungszeitraum vom 3 Tagen zu verabreichen ist.

4. Die Verwendung gemäß einem der 1 bis 3, wobei die Gesamtdosis intravenös zu verabreichen ist.

5. Die Verwendung gemäß 4, wobei zunächst ein Anteil von 20 bis 50 % der Gesamtdosis bei Aufnahme der Behandlung als Bolus zu verabreichen ist, während der restliche Anteil kontinuierlich über den Behandlungszeitraum zu verabreichen ist.

6. Die Verwendung gemäß 5, wobei der als Bolus zu verabreichende Anteil ein Drittel der Gesamtdosis ausmacht.

7. Verfahren zur Identifikation von Schlaganfallpatienten, die ansprechen auf eine Behandlung umfassend die Verabreichung von G-CSF, und umfassend die Schritte

a) DWI-busierte Bestimmung des Infarktvolumens, und
b) Identifikation der Patienten mit einem Infarktvolumen von min. 16 cm$^3$ als Kandidaten für besagte Behandlung.

8. Verfahren zur Identifikation von Schlaganfallpatienten, die ansprechen auf eine Behandlung umfassend die Verabreichung von G-CSF, und umfassend die Schritte

a) DWI-basierte Bestimmung des Infarktvolumens, und

b) Identifikation der Patienten mit einem Infarktvolumen mit Beteiligung des corticalen Hirngewebes als Kandidaten für besagte Behandlung.

9. Das Verfahren gemäß 7 oder 8, wobei die Verabreichung von G-CSF erfolgt, wie in einem der 1 bis 6 definiert.

10. Die Verwendung gemäß einem der 1 bis 6, wobei die zu behandelnden Schlaganfallpatienten einen Infarkt mit corticaler Beteiligung aufweisen.

11. Die Verwendung nach . 10, wobei die zu behandelnden Schlaganfallpatienten vor der Behandlung darauf getestet wurden, ob sie einen Infarkt mit corticaler Beteiligung aufweisen.

12. Die Verwendung gemäß einem der 1 bis 6, wobei die zu behandelnden Patienten einen Schlaganfall mit einem initialen DWI-bestimmten Infarktvolumen von min. 16 $cm^3$ aufweisen.

13. Die Verwendung gemäß 12, wobei die zu behandelnden Patienten einen Schlaganfall mit einem initialen DWI-bestimmten Infarktvolumen von min. 25 $cm^3$ aufweisen.

14. Die Verwendung gemäß 13, wobei die zu behandelnden Patienten einen Schlaganfall, mit einem initialen DWI-bestimmten Infarktvolumen von min. 50 $cm^3$ aufweisen.

15. Die Verwendung nach einem der 12 bis 14, wobei die Patienten vor der Behandlung darauf getestet werden, ob sie einen Schlaganfall mit einem initialen DW1-bestimmten Infarktvolumen von dem jeweiligen Mindestvolumen aufweisen.

16. Verfahren zur Behandlung eines Patienten mit cerebralem Schlaganfall, umfassend die Verabreichung von G-CSF in einer Gesamtdosis wie in 1 oder 2 definiert.

17. Verfahren zur Behandlung eines Patienten mit cerebralem Schlaganfall, umfassend die Verabreichung von G-CSF in einer Gesamtdosis wie in 1 oder 2 definiert, wobei besagter Patient ein initiales Infarktvolumen aufweist, wie es in einem der 10 oder 12 bis 14 definiert ist.

**Patentansprüche**

1. G-CSF zur Verwendung in einem Verfahren zur Behandlung von Schlaganfall beim menschlichen Patienten, wobei das G-CSF dem Patienten in einer Gesamtdosis von 80 bis 150 μg pro kg Körpergewicht über einen Behandlungszeitraum von 2 bis 7 Tagen zu verabreichen ist.

2. Verwendung von G-CSF für die Herstellung eines Arzneimittels zur Behandlung von Schlaganfall beim menschlichen Patienten, wobei das G-CSF dem Patienten in einer Gesamtdosis von 80 bis 150 μg pro kg Körpergewicht über einen Behandlungszeitraum von 2 bis 7 Tagen zu verabreichen ist.

3. G-CSF zur Verwendung gemäß Anspruch 1 oder Verwendung gemäß Anspruch 2, wobei die zu verabreichende Gesamtdosis zwischen 120 und 150 μg pro kg Körpergewicht liegt.

4. G-CSF zur Verwendung oder Verwendung gemäß einem der Ansprüche 1 bis 3, wobei die Gesamtdosis über einen Behandlungszeitraum von 3 Tagen zu verabreichen ist.

5. G-CSF zur Verwendung oder Verwendung gemäß einem der Ansprüche 1 bis 4, wobei die Gesamtdosis intravenös zu verabreichen ist.

6. G-CSF zur Verwendung oder Verwendung gemäß Anspruch 5, wobei zunächst ein Anteil von 20 bis 50 % der Gesamtdosis bei Aufnahme der Behandlung als Bolus zu verabreichen ist, während der restliche Anteil kontinuierlich über den Behandlungszeitraum zu verabreichen ist.

7. G-CSF zur Verwendung oder Verwendung gemäß Anspruch 6, wobei der als Bolus zu verabreichende Anteil ein Drittel der Gesamtdosis ausmacht.

**8.** G-CSF zur Verwendung oder Verwendung gemäß einem der Ansprüche 1 bis 7, wobei die zu behandelnden Schlaganfallpatienten einen Infarkt mit corticaler Beteiligung aufweisen.

**9.** G-CSF zur Verwendung oder Verwendung nach Anspruch 8, wobei die zu behandelnden Schlaganfallpatienten vor der Behandlung darauf getestet wurden, ob sie einen Infarkt mit corticaler Beteiligung aufweisen.

**10.** G-CSF zur Verwendung oder Verwendung gemäß einem der Ansprüche 1 bis 7, wobei die zu behandelnden Patienten einen Schlaganfall mit einem initialen DWI-bestimmten Infarktvolumen von min. 16 cm$^3$ aufweisen.

**11.** G-CSF zur Verwendung oder Verwendung gemäß Anspruch 10, wobei die zu behandelnden Patienten einen Schlaganfall mit einem initialen DWI-bestimmten Infarktvolumen von min. 25 cm$^3$ aufweisen.

**12.** G-CSF zur Verwendung oder Verwendung gemäß Anspruch 11, wobei die zu behandelnden Patienten einen Schlaganfall mit einem initialen DWI-bestimmten Infarktvolumen von min. 50 cm$^3$ aufweisen.

**13.** G-CSF zur Verwendung oder Verwendung nach einem der Ansprüche 10 bis 12, wobei die Patienten vor der Behandlung darauf getestet werden, ob sie einen Schlaganfall mit einem initialen DWI-bestimmten Infarktvolumen von dem jeweiligen Mindestvolumen aufweisen.

**14.** G-CSF zur Verwendung in einem Verfahren zur Behandlung eines Patienten mit cerebralem Schlaganfall, umfassend die Verabreichung von G-CSF in einer Gesamtdosis wie in Anspruch 2 oder 3 definiert.

**15.** G-CSF zur Verwendung nach Anspruch 14, wobei besagter Patient ein initiales Infarktvolumen aufweist, wie es in einem der Ansprüche 8 oder 10 bis 12 definiert ist.

Fig. 1:

Fig. 2:

Fig. 3:

Europäisches
Patentamt

European
Patent Office

Office européen
des brevets

**EUROPÄISCHER RECHERCHENBERICHT**

Nummer der Anmeldung

EP 11 00 8516

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | SCHABITZ W -R ET AL: "New targets for established proteins: exploring G-CSF for the treatment of stroke", TRENDS IN PHARMACOLOGICAL SCIENCES 200704 GB, Bd. 28, Nr. 4, April 2007 (2007-04), Seiten 157-161, XP022006216, ISSN: 0165-6147 * das ganze Dokument * ----- | 1,2,4-15 | INV. A61K38/19 A61P9/10 |
| Y,D | WO 02/099081 A (QUARK BIOTECH INC [US]; CHAJUT AYELET [IL]) 12. Dezember 2002 (2002-12-12) * Ansprüche; Beispiel 2 * ----- | 1,2,4-15 | |
| Y | ZHAO LI-RU ET AL: "Brain repair by hematopoietic growth factors in a rat model of stroke", STROKE, Bd. 38, Nr. 9, September 2007 (2007-09), Seiten 2584-2591, XP002482802, ISSN: 0039-2499 * Seite 2585, linke Spalte, Absatz 4 * ----- | 1,2,4-15 | |
| Y | SCHNEIDER ARMIN ET AL: "An extended window of opportunity for G-CSF treatment in cerebral ischemia", BMC BIOLOGY, Bd. 4, Oktober 2006 (2006-10), XP021024963, * Zusammenfassung * ----- -/-- | 1,2,4-15 | |

RECHERCHIERTE
SACHGEBIETE (IPC)

A61K
A61P

Der vorliegende Recherchenbericht wurde für alle Patentansprüche erstellt

| Recherchenort | Abschlußdatum der Recherche | Prüfer |
|---|---|---|
| München | 16. Dezember 2011 | Winger, Rudolf |

EPO FORM 1503 03.82 (P04C03)

Europäisches
Patentamt

European
Patent Office

Office européen
des brevets

# EUROPÄISCHER RECHERCHENBERICHT

Nummer der Anmeldung

EP 11 00 8516

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | KAWADA HIROSHI ET AL: "Administration of hematopoietic cytokines in the subacute phase after cerebral infarction is effective for functional recovery facilitating proliferation of intrinsic neural stem/progenitor cells and transition of bone marrow-derived neuronal cells", CIRCULATION, Bd. 113, Nr. 5, Februar 2006 (2006-02), Seiten 701-710, XP002482803, ISSN: 0009-7322 * Seite 703, linke Spalte, Absatz 1 * ----- | 1,2,4-15 | |
| Y | BORLONGAN CESAR V ET AL: "New hope for stroke patients: mobilization of endogenous stem cells.", CMAJ : CANADIAN MEDICAL ASSOCIATION JOURNAL = JOURNAL DE L'ASSOCIATION MEDICALE CANADIENNE 28 MAR 2006, Bd. 174, Nr. 7, 28. März 2006 (2006-03-28) , Seiten 954-955, XP002482804, ISSN: 1488-2329 * Seite 955, linke Spalte, Absatz 2 * ----- | 1,2,4-15 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 16. Dezember 2011 | Winger, Rudolf |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...............................................................................
& : Mitglied der gleichen Patentfamilie, übereinstimmendes
    Dokument

EPO FORM 1503 03.82 (P04C03)

# EP 2 412 381 A1

**ANHANG ZUM EUROPÄISCHEN RECHERCHENBERICHT
ÜBER DIE EUROPÄISCHE PATENTANMELDUNG NR.**

EP 11 00 8516

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-12-2011

| Im Recherchenbericht angeführtes Patentdokument | Datum der Veröffentlichung | Mitglied(er) der Patentfamilie | Datum der Veröffentlichung |
|---|---|---|---|
| WO 02099081 A | 12-12-2002 | AU 2002316201 A1<br>WO 02099081 A2 | 16-12-2002<br>12-12-2002 |

Für nähere Einzelheiten zu diesem Anhang : siehe Amtsblatt des Europäischen Patentamts, Nr.12/82

13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 03537151 B
- EP 1019082 A
- EP 1465653 A
- DE 10033219

**In der Beschreibung aufgeführte Nicht-Patentliteratur**

- **LEKER ; SHOLZAMI.** *Brain Res. Rev.,* 2002, vol. 39, 55-73
- **BEAULIEU et al.** *Ann Neurol,* 1999, vol. 46, 558-578
- **WU et al.** *Stroke,* 2001, vol. 32, 933-942
- **BEAULIEU et al.** *Ann Neurol,* 1999, vol. 46, 568-578
- **METCALF.** *Cancer,* 1990, vol. 65, 2185-2194
- **PIMENTEL.** *Ann. Clin. Lab. Sc.,* 1990, vol. 20, 36-55
- **NAGATA.** *BioEssays,* 1989, vol. 10, 113-117
- **BUSCHMANN et al.** *Circulation,* 2003, vol. 108, 610-615
- **SCHABITZ et al.** *Stroke,* 2003, vol. 34, 745-751
- **SCHNEIDER et al.** *J Clin Invest,* 2005, vol. 115, 2083-2098
- **EMERICH et al.** *Clin Pharmacokinet,* 2001, vol. 40, 105-123
- **SIEGAL et al.** *Clin Pharmacokinet,* 2002, vol. 41, 171-186